(19) Europäisches Patentamt
European Patent Office
Office européen des brevets

(11) Veröffentlichungsnummer: **0 103 142**
**B1**

(12) # EUROPÄISCHE PATENTSCHRIFT

(45) Veröffentlichungstag der Patentschrift: **22.04.87**

(51) Int. Cl.⁴: **C 07 D 513/04**, A 61 K 31/54 // (C07D513/04, 333:00, 279:00)

(21) Anmeldenummer: **83107677.3**

(22) Anmeldetag: **04.08.83**

(54) **Thieno(2,3-e)-1,2-thiazin-Derivate.**

(30) Priorität: **09.09.82 CH 5377/82**

(43) Veröffentlichungstag der Anmeldung: **21.03.84 Patentblatt 84/12**

(45) Bekanntmachung des Hinweises auf die Patenterteilung: **22.04.87 Patentblatt 87/17**

(84) Benannte Vertragsstaaten: **AT BE CH DE FR GB IT LI LU NL SE**

(56) Entgegenhaltungen:
**EP - A - 0 001 113**
**DE - A - 2 537 070**

**J. of Medicinal and Pharmaceutical Chemistry, Vol. 1, No. 2 (1959), p. 121-33.**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Pfister, Rudolf, Dr., Neubadstrasse 128, CH-4054 Basel (CH)**
Erfinder: **Zeller, Paul, Dr., Rosenbergweg 54, CH-4123 Allschwil (CH)**
Erfinder: **Binder, Dieter, Prof., Sieveringerstrasse 207, A-1190 Wien (AT)**
Erfinder: **Hromatka, Otto, Prof., Starkfriedgasse 89, Wien (AT)**

(74) Vertreter: **Bertschinger, Christoph, Dr. et al, Grenzacherstrasse 124 P.O. Box 3255, CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft Thiazinderivate. Im speziellen betrifft sie 4-Hydroxy-2-methyl-3-(2-pyridylcarbamoyl)-6-trifluormethyl-2H-thieno[2,3-e]-1,2-thiazin-1,1-dioxid der Formel I

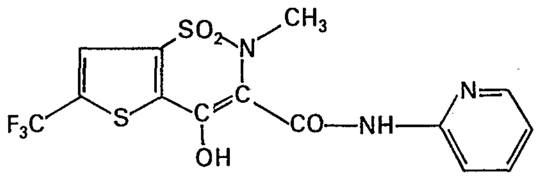

(I)

und pharmazeutisch annehmbare Salze davon.

Diese Stoffe sind neu und zeichnen sich durch wertvolle pharmakodynamische Eigenschaften aus.

Gegenstand der Erfindung sind die Verbindung der Formel I und pharmazeutisch annehmbare Salze davon als solche und als pharmazeutische Wirkstoffe, die Herstellung dieser Stoffe und Zwischenprodukte für deren Herstellung, ferner Arzneimittel, enthaltend einen solchen Stoff, und die Herstellung solcher Arzneimittel, sowie die Verbindung der Formel I oder ihrer pharmazeutisch annehmbaren Salze bei der Bekämpfung bzw. Verhütung von Krankheiten. In EP-A 0 001 113 und DE-A 2 537 070 werden u.a. die entsprechende 6-Chlorverbindung bzw. die entsprechende am Thiophenring unsubstituierte Verbindung beschrieben.

Der in dieser Beschreibung verwendete Ausdruck «($C_1$–$C_7$)-Alkyl» bezeichnet geradkettige oder verzweigte gesättigte Kohlenwasserstoffgruppen mit 1–7 Kohlenstoffatomen, wie Methyl oder Äthyl.

Die Verbindung der Formel I und ihre pharmazeutisch annehmbaren Salze können erfindungsgemäss dadurch hergestellt werden, dass man eine Verbindung der allgemeinen Formel II

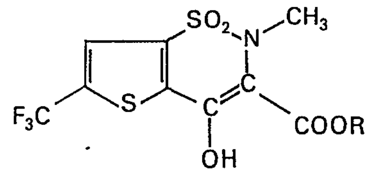

(II)

worin R ($C_1$–$C_7$)-Alkyl bedeutet, mit 2-Aminopyridin umsetzt, und erwünschtenfalls die so erhaltene Verbindung der Formel I in ein pharmazeutisch annehmbares Salz überführt.

Die erfindungsgemässe Umsetzung kann in An- oder Abwesenheit eines inerten Lösungsmittels erfolgen. Als Lösungsmittel eignen sich Kohlenwasserstoffe, wie Benzol, Toluol oder Xylol, halogenierte Kohlenwasserstoffe, wie Chloroform, Chlorbenzol, Methylenchlorid oder Tetrachlorkohlenstoff, oder Dimethylformamid oder Dioxan. Die Umsetzung erfolgt vorzugsweise unter Erwärmen, wobei Schmelz- bzw. Rückflusstemperatur des Reaktionsgemisches besonders bevorzugt ist.

Die Verbindungen der allgemeinen Formel II sind neu und ebenfalls Gegenstand der Erfindung; sie können beispielsweise ausgehend von Verbindungen der allgemeinen Formel III

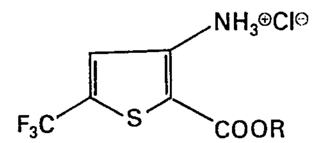

(III)

worin R obige Bedeutung besitzt,

welche einer an sich bekannten Stoffklasse angehören, gemäss dem nachfolgenden Formelschema und dem weiter unten folgenden spezifischen Beispiel hergestellt werden.

Reaktionsschema

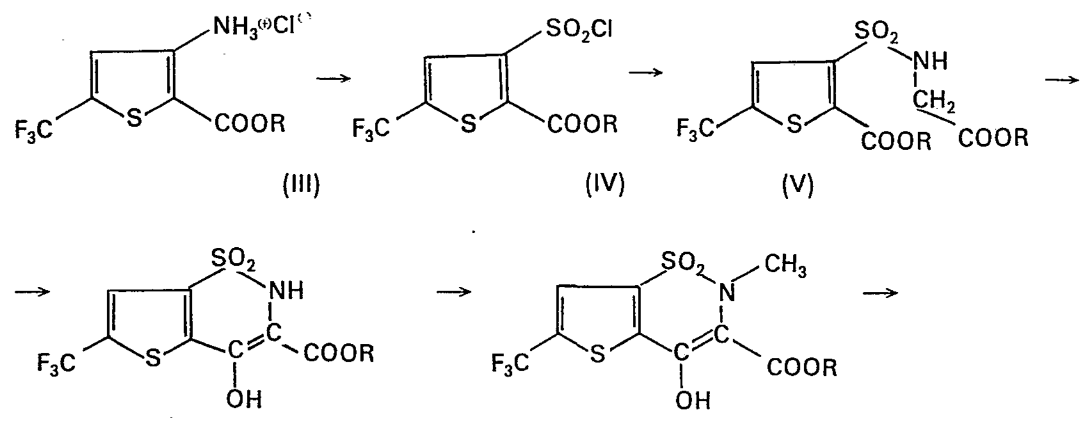

(III) (IV) (V) (VI) (II)

Die Verbindung der Formel I besitzt ein acides Wasserstoffatom und kann mit Basen pharmazeutisch annehmbare Salze bilden. Als derartige Salze eignen sich z.B. Alkalimetallsalze, wie das Lithium-, Natrium- oder Kaliumsalz; Erdalkalimetallsalze, wie Magnesium- oder Calciumsalze; sowie Salze mit Aminen, wie Triäthanolamin, Diäthylaminoäthanol, Triäthylamin, Trimethylamin

oder Diäthylamin. Die Verbindung der Formel I kann mit starken Säuren auch pharmazeutisch annehmbare Säureadditionssalze bilden, wozu insbesondere Mineralsäuren, wie Salzsäure in Frage kommen.

Die Verbindung der Formel I sowie deren pharmazeutisch annehmbare Salze entfalten antiinflammatorische, analgetische und antirheumatische Wirkungen. Diese wertvollen pharmakologischen Eigenschaften können unter Verwendung von Standardmethoden bestimmt werden, beispielsweise im bekannten Carrageenin-Pfotenoedemtest an der Ratte. In diesem Test wird in der rechten Hinterpfote der Ratte durch intradermale Injektion von 0,1 ml einer 5-proz. Carrageenin-Lösung eine akute lokale Entzündung erzeugt. Die zu untersuchende Substanz wird auf oralem Wege verabreicht, und der Durchmesser der Pfote wird in mm (als Ausdruck der Heftigkeit der Entzündung) gemessen.

Nach der Carageenin-Injektion wird die zu untersuchende Substanz verabreicht, und 2, 3 und 4 Stunden nach der Carrageenin-Injektion wird der Pfoten-Durchmesser gemessen. Der oedemhemmende Effekt wird auf Grund der Differenz der Oedemintensität zwischen unbehandelten und mit der zu untersuchenden Substanz behandelten Tieren ermittelt. Die $ED_{30}$ ist diejenige Dosis, welche notwendig ist, um den Durchmesser der durch die Carrageenin-Injektion entzündeten Pfote um 30% zu verkleinern. Für die Verbindung der Formel I wurde eine $ED_{30}$ von 1,91 mg/kg p.o. ermittelt.

Die Verbindung der Formel I besitzt qualitativ eine ähnliche Wirkung wie Phenylbutazon, welches für seine therapeutische Verwendung und Eigenschaften bekannt ist.

Die Verbindung der Formel I sowie deren pharmazeutisch annehmbare Salze können als Heilmittel, z.B. in Form pharmazeutischer Präparate Verwendung finden, welche sie in Mischung mit einem für die enterale oder parenterale Applikation geeigneten, pharmazeutischen, organischen oder anorganischen inerten Trägermaterial, wie z.B. Wasser, Gelatine, Gummi arabicum, Milchzucker, Stärke, Magnesiumstearat, Talk, pflanzliche Öle, Polyalkylenglykole, Vaseline usw. enthalten. Die pharmazeutischen Präparate können in fester Form, z.B. als Tabletten, Dragées, Suppositorien, Kapseln, in halbfester Form, z.B. als Salben, oder in flüssiger Form, z.B. als Lösungen, Suspensionen oder Emulsionen, vorliegen. Gegebenenfalls sind sie sterilisiert und/oder enthalten Hilfsstoffe, wie Konservierungs-, Stabilisierungs- oder Emulgiermittel, Salze zur Veränderung des osmotischen Druckes oder Puffer. Sie können auch noch andere therapeutisch wertvolle Stoffe enthalten.

Wie eingangs erwähnt, kann man die Verbindung der Formel I und ihre pharmazeutisch annehmbaren Salze erfindungsgemäss bei der Bekämpfung bzw. Verhütung von Krankheiten verwenden, insbesondere bei der Bekämpfung bzw. Verhütung von Entzündungen, Schmerzen und rheumatischen Erkrankungen. Die Dosierung der Verbindung der Formel I und ihrer pharmazeutisch annehmbaren Salze kann innerhalb ziemlich weiter Grenzen variieren und ist natürlich in jedem einzelnen Fall den individuellen Gegebenheiten anzupassen. Im allgemeinen dürfte beim Menschen eine orale Tagesdosis von etwa 5 bis etwa 100 mg, vorzugsweise etwa 10 bis etwa 30 mg, angemessen sein.

Wie eingangs erwähnt, sind Arzneimittel, enthaltend die Verbindung der Formel I oder ein pharmazeutisch annehmbares Salz davon, ebenfalls Gegenstand der Erfindung, weiterhin auch ein Verfahren zur Herstellung solcher Arzneimittel, welches dadurch gekennzeichnet ist, dass man die Verbindung der Formel I oder ein pharmazeutisch annehmbares Salz davon und gegebenenfalls einen oder mehrere andere therapeutisch wertvolle Stoffe in eine galenische Darreichungsform bringt.

Die nachfolgenden Beispiele, in welchen alle Temperaturen in Celsiusgraden angegeben sind, erläutern die Erfindung.

Beispiel

a) Man stellt eine Lösung von 172 g rohem 3-Amino-5-trifluormethyl-thiopen-2-carbonsäureäthylester-hydrochlorid [Monatshefte f. Chemie 105, 132 (1974)] in wenig Wasser mit Natriumhydrogencarbonat alkalisch und extrahiert das ausgefallene Material dreimal mit je 300 ml Methylenchlorid. Die vereinigten organischen Auszüge werden über Natriumsulfat getrocknet und eingedampft.

Man versetzt die so erhaltene freie Base (102 g) mit einer Lösung von 2 g Natrium in 800 ml absolutem Methanol und erhitzt die erhaltene Lösung während 40 Minuten unter Rückfluss zum Sieden. Nach dem Abkühlen gibt man 6 ml Eisessig dazu, entfernt weitgehend das Methanol im Vakuum und verteilt den Rückstand zwischen Methylenchlorid und gesättigter Natriumbicarbonatlösung. Die organische Phase wird abgetrennt, über Natriumsulfat getrocknet und eingedampft. Der ölige Rückstand wird zur Entfernung restlichen Methylenchlorids noch zweimal mit etwas Benzol abgedampft und dann in 600 ml absolutem Benzol aufgenommen. Man versetzt die Lösung mit drei Teelöffel Aktivkohle, saugt ab und leitet bis zur vollständigen Ausfällung trockenes Chlorwasserstoff-Gas ein. Das farblose kristalline 3-Amino-5-trifluormethyl-thiopen-2-carbonsäuremethylester-hydro-chlorid wird abgesaugt, mit Benzol gewaschen und an der Luft getrocknet.

b) 5,60 g (0,0217 Mol) 3-Amino-5-trifluormethyl-thiophen-2-carbonsäuremethylester-hydrochlorid werden bei −2° portionsweise und unter Rühren in 11,3 ml konzentrierte Salzsäure eingetragen, wobei ein dicker Brei entsteht. Dann wird bei −5 bis 0° eine Lösung von 1,51 g Natriumnitrit in 2,7 ml Wasser während 1,5 Stunden unter die Oberfläche der Suspension eingeleitet, wobei gegen Ende der Zugabe die Reaktionsmischung dünnflüssig wird. Es wird noch 1 Stunde bei −3° gerührt, worauf man die Lösung des erhaltenen Diazoniumsalzes auf einmal zu einer Mischung von 2,4 ml gesättigter Kupfer (II)-chloridlösung und 38 ml 30proz. Schwefeldioxidlösung in Eisessig gibt, wobei sofort heftiges Aufschäumen eintritt. Man rührt noch 1 Stunde bei Raumtemperatur, giesst dann auf 70 ml Eiswasser und extrahiert das ausgefallene Öl fünfmal mit je 50 ml Methylenchlorid. Die vereinigten organischen Auszüge werden dreimal mit je 25 ml Wasser gewaschen, über Natriumsulfat getrocknet und im Vakuum eingedampft. Der ölige Rückstand, bestehend aus 3-Chlorsulfonyl-5-trifluormethyl-thiophen-2-carbonsäure-methylester, wird ohne weitere Reinigung in die nächste Stufe eingesetzt.

c) Man löst 103,8 g (0,336 Mol) 3-Chlorsulfonyl-5-trifluormethyl-thiophen-2-carbonsäure-methylester in 600 ml Chloroform, tropft 68,05 g (0,765 Mol) frisch destillierten Glycinmethylester so dazu, dass die Temperatur von 30° nicht überschritten wird, rührt noch 0,5 Stunden bei Raumtemperatur und schüttelt die Reaktionsmischung dann dreimal mit je 200 ml 1N-Salzsäure aus. Die organische Phase wird über Natriumsulfat getrocknet und eingedampft. Der ölige Rückstand wird mit 175 ml Diisopropyläther gerührt, wobei man 3-[(Methoxycarbonylmethyl)sulfamoyl]-5-trifluormethyl-thiophen-2-carbonsäure-methylester in Form farbloser Kristalle erhält, die abgesaugt und mit Diisopropyläther gewaschen werden. Nach Umkristallisieren aus Diisopropyläther schmilzt das Produkt bei 76–78°.

d) Eine Lösung von 82,8 g (0,229 Mol) 3-[(Methoxycarbonylmethyl)sulfamoyl]-5-trifluormethyl-thiophen-2-carbonsäure-methylester in 1,7 l absolutem Tetrahydrofuran wird auf 3° abgekühlt und bei dieser Temperatur während 20 Minuten portionsweise mit insgesamt 62,9 g (0,562 Mol) Kaliumtert.-butylat versetzt. Danach entfernt man das Kühlbad, rührt den dickflüssigen roten Brei 2 Stunden bei Raumtemperatur und destilliert dann im Vakuum bei höchstens 30° möglichst viel Tetrahydrofuran ab. Den pulverigen Rückstand nimmt man in 1,4 l Eiswasser auf, versetzt unter Rühren mit 10 g Aktivkohle und saugt über Kieselgur ab. Das Filtrat wird mit ca. 185 ml 3N-Salzsäure bei maximal 5° auf pH 2–3 gebracht. Man rührt 0,5 Stunden, wobei das zunächst ölig ausfallende Rohprodukt kristallisiert (nach Zugabe von etwas Äther). Das ausgefallene Rohprodukt wird abgesaugt, mit Wasser neutralgewaschen, bei 50° über Nacht im Vakuum getrocknet und aus Methanol umkristallisiert. Man erhält 4-Hydroxy-3-methoxycarbonyl-6-trifluor-methyl-2H-thieno[2,3-e]-1,2-thiazin-1,1-dioxid vom Schmelzpunkt 168–170°.

e) Man versetzt eine auf −2° abgekühlte Lösung von 5,65 g (0,0172 Mol) 4-Hydroxy-3-methoxycarbonyl-6-trifluor-methyl-2H-thieno[2,3-e]-1,2-thiazin-1,1-dioxid in 62 ml absolutem Dimethylformamid unter starkem Stickstoffstrom portionenweise mit 0,87 g (0,0362 Mol) Natriumhydrid (mit Benzol gewaschen), rührt noch 1,5 Stunden bei 5°, gibt dann auf einmal 1,49 ml (0,024 Mol) Methyljodid dazu, wobei die Temperatur auf ca. 15° steigt, rührt noch 1,5 Stunden bei Raumtemperatur und dampft dann im Vakuum ein. Den Rückstand verteilt man zwischen 2N-Salzsäure und Methylenchlorid, schüttelt die wässrige Phase noch dreimal mit Methylenchlorid aus, trocknet die vereinigten organischen Auszüge über Natriumsulfat und dampft ein. Man digeriert den braunen kristallinen Rückstand mit ca. 50 ml Aethanol, saugt die fast farblosen Kristalle ab und wäscht sie mit Methanol. Man erhält, nach Umkristallisieren aus Aethanol, 4-Hydroxy-3-methoxycarbonyl-2-methyl-6-trifluormethyl-2H-thieno[2,3-e]-1,2-thiazin-1,1-dioxid vom Schmelzpunkt 178–180°.

f) 4,25 g (0,0124 Mol) 4-Hydroxy-3-methoxycarbonyl-2-methyl-6-trifluormethyl-2H-thieno[2,3-e]-1,2-thiazin-1,1-dioxid werden unter Rühren zusammen mit 1,54 g (0,0163 Mol) 2-Aminopyridin in 192 ml Xylol 5 Stunden unter Rückfluss zum Sieden erhitzt, wobei man langsam Lösungsmittel abdestilliert und durch frisches Xylol ergänzt. Man dampft anschliessend im Vakuum ein, digeriert den halbkristallinen Rückstand in ca. 40 ml Dioxan, saugt dann die erhaltenen gelben Kristalle ab und wäscht sie mit Dioxan. Man verteilt das Rohprodukt zwischen 30 ml Methylenchlorid und 40 ml Wasser, das 3 ml 10proz. Natronlauge enthält, trennt die Phasen, versetzt die wässrige Phase mit 0,5 g Aktivkohle, filtriert durch Kieselgur, erwärmt das Filtrat auf 30° und stellt es mit ca. 2,7 ml 3N-Salzsäure (unter Rühren während 1 Stunde) auf pH 2 ein. Die ausgefallenen gelben, feinen Kristalle werden abgesaugt, reichlich mit Wasser gewaschen und während 8 Stunden im Hochvakuum (0,1 mm Hg) bei 105° getrocknet. Man erhält 4-Hydroxy-2-methyl-3-(2-pyridylcarbamoyl)-6-trifluormethyl-2H-thieno[2,3-e]-1,2-thiazin-1,1-dioxid vom Schmelzpunkt 230° (Zersetzung).

Beispiel A

Es werden in üblicher Weise Suppositorien folgender Zusammensetzung hergestellt:

| | |
|---|---|
| 4-Hydroxy-2-methyl-3-(2-pyridylcarbamoyl)-6-trifluormethyl-2H-thieno[2,3-e]-1,2-thiazin-1,1-dioxid | 0,005 g |
| Hydriertes Kokosnussöl | 1,250 g |
| Carnaubawachs | 0,045 g |

Beispiel B

Es werden in üblicher Weise Tabletten folgender Zusammensetzung hergestellt:

| | Pro Tablette |
|---|---|
| 4-Hydroxy-2-methyl-3-(2-pyridylcarbamoyl)-6-trifluormethyl-2H-thieno[2,3-e]-1,2-thiazin-1,1-dioxid | 5,00 mg |
| Lactose | 84,50 mg |
| Maisstärke | 10,00 mg |
| Magnesiumstearat | 0,50 mg |

Beispiel C

Es werden in üblicher Weise Kapseln folgender Zusammensetzung hergestellt:

| | Pro Kapsel |
|---|---|
| 4-Hydroxy-2-methyl-3-(2-pyridylcarbamoyl)-6-trifluormethyl-2H-thieno[2,3-e]-1,2-thiazin-1,1-dioxid | 10 mg |

| | |
|---|---|
| Lactose | 165 mg |
| Maisstärke | 30 mg |
| Talk | 5 mg |
| Gesamtgewicht | 210 mg |

**Patentansprüche für die Vertragsstaaten: BE, CH, DE, FR, GB, IT, LI, LU, NL, SE**

1. 4-Hydroxy-2-methyl-3-(2-pyridylcarbamoyl)-6-trifluor-methyl-2H-thieno[2,3-e]-1,2-thiazin-1,1-dioxid der Formel I

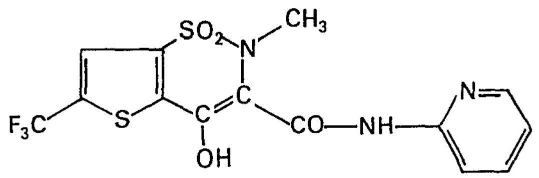

(I)

und pharmazeutisch annehmbare Salze davon.

2. Verbindungen der allgemeinen Formel II

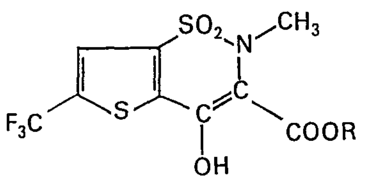

(II)

worin R ($C_1$–$C_7$)-Alkyl bedeutet.

3. Verbindungen gemäss Anspruch 1 als pharmazeutische Wirkstoffe.

4. Verbindungen gemäss Anspruch 1 als entzündungshemmende, schmerzstillende und antirheumatische Wirkstoffe.

5. Verfahren zur Herstellung von 4-Hydroxy-2-methyl-3-(2-pyridylcarbamoyl)-6-trifluormethyl-2H-thieno[2,3-e]-1,2-thiazin-1,1-dioxid und pharmazeutisch annehmbaren Salzen davon, dadurch gekennzeichnet, dass man eine Verbindung der allgemeinen Formel II

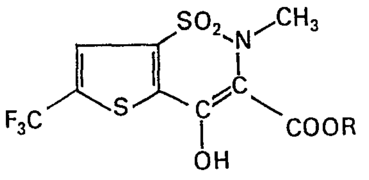

(II)

worin R ($C_1$–$C_7$)-Alkyl bedeutet, mit 2-Aminopyridin umsetzt und erwünschtenfalls die so erhaltene Verbindung in ein pharmazeutisch annehmbares Salz überführt.

6. Arzneimittel, enthaltend 4-Hydroxy-2-methyl-3-(2-pyridylcarbamoyl)-6-trifluormethyl-2H-thieno[2,3-e]-1,2-thiazin-1,1-dioxid oder ein pharmazeutisch annehmbares Salz davon.

7. Entzündungshemmende, schmerzstillende und antirheumatische Mittel, enthaltend 4-Hydroxy-2-methyl-3-(2-pyridylcarbamoyl)-6-trifluormethyl-2H-thieno[2,3-e]-1,2-thiazin-1,1-dioxid oder ein pharmazeutisch annehmbares Salz davon.

**Patentanspruch für den Vertragsstaat: AT**

1. Verfahren zur Herstellung von 4-Hydroxy-2-methyl-3-(2-pyridylcarbamoyl)-6-trifluormethyl-2H-thieno[2,3-e]-1,2-thiazin-1,1-dioxid der Formel I

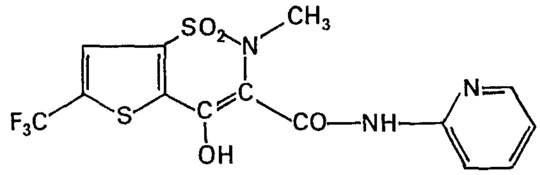

(I)

und pharmazeutisch annehmbaren Salzen davon, dadurch gekennzeichnet, dass man eine Verbindung der allgemeinen Formel II

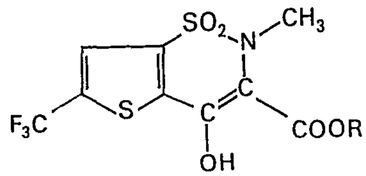

(II)

worin R ($C_1$–$C_7$)-Alkyl bedeutet, mit 2-Aminopyridin umsetzt, und erwünschtenfalls die so erhaltene Verbindung in ein pharmazeutisch annehmbares Salz überführt.

**Patentansprüche für die Vertragsstaaten: BE, CH, DE, FR, GB, IT, LI, LU, NL, SE**

1. 4-Hydroxy-2-methyl-3-(2-pyridylcarbamoyl)-6-trifluor-methyl-2H-thieno[2,3-e]-1,2-thiazin-1,1-dioxid der Formel I

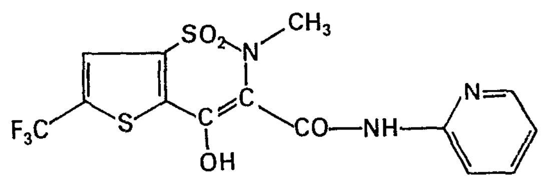

(I)

and pharmaceutically acceptable salts thereof.

2. Compounds of general formula II

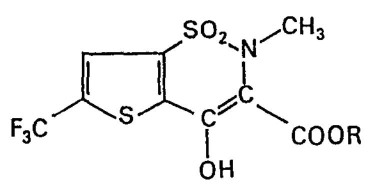

(II)

wherein R signifies ($C_1$–$C_7$)-alkyl.

3. Compounds in accordance with claim 1 as pharmaceutically active substances.

4. Compounds in accordance with claim 1 as antiinflammatory, analgesic and antirheumatic active substances.

5. A process for the manufacture of 4-hydroxy-2-methyl-3-(2-pyridylcarbamoyl)-6-trifluoromethyl-2H-thieno[2,3-e]-1,2-thiazine 1,1-dioxide and pharmaceutically acceptable salts thereof, characterized by reacting a compound of general formula II

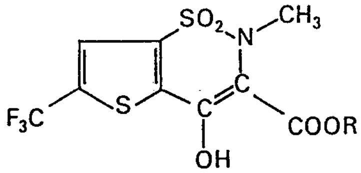

(II)

wherein R signifies ($C_1$–$C_7$)-alkyl,

with 2-aminopyridine and, if desired, converting the thus-obtained compound into a pharmaceutically acceptable salt.

6. A medicament containing 4-hydroxy-2-methyl-3-(2-pyridylcarbamoyl)-6-trifluoromethyl-2H-thieno[2,3-e]-1,2-thiazine 1,1-dioxide or a pharmaceutically acceptable salt thereof.

7. An antiinflammatory, analgesic and antirheumatic medicament containing 4-hydroxy-2-methyl-3-(2-pyridylcarbamoyl)-6-trifluoromethyl-2H-thieno[2,3-e]-1,2-thiazine 1,1-dioxide or a pharmaceutically acceptable salt thereof.

**Claim for the Contracting State: AT**

1. A process for the manufacture of 4-hydroxy-2-methyl-3-(pyridylcarbamoyl)-6-trifluoromethyl-2H-thieno[2,3-e]-1,2-thiazine 1,1-dioxide of formula I

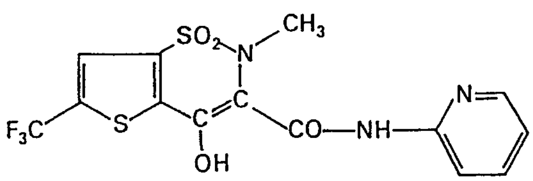

(I)

and pharmaceutically acceptable salts thereof, characterized by reacting a compound of general formula II

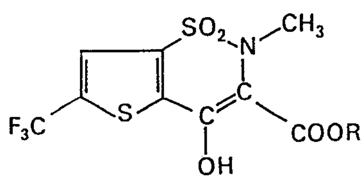

(II)

wherein R signifies ($C_1$–$C_7$)-alkyl, with 2-aminopyridine and, if desired, converting the thus-obtained compound into a pharmaceutically acceptable salt.

**Revendications pour les Etats contractants: BE, CH, DE, FR, GB, IT, LI, LU, NL, SE**

1. Le 1,1-dioxyde de la 4-hydroxy-2-méthyl-3-(2-pyridylcarbamoyl)-6-trifluorométhyl-2H-thiéno[2,3-e]-1,2-thiazine de formule I

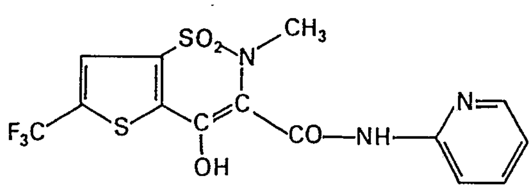

(I)

et ses sels acceptables pour l'usage pharmaceutique.

2. Composés de formule générale II

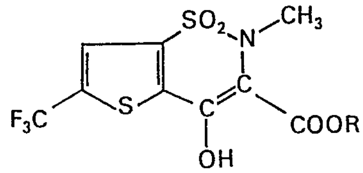

(II)

dans laquelle R représente un groupe alkyle en $C_1$–$C_7$.

3. Composés selon la revendication 1, en tant que substances actives pharmaceutiques.

4. Composés selon la revendication 1, en tant que substances actives anti-inflammatoires, analgésiques et anti-rhumatismales.

5. Procédé de préparation du 1,1-dioxyde de la 4-hydroxy-2-méthyl-3-(2-pyridylcarbamoyl)-6-trifluorométhyl-2H-thiéno[2,3-e]-1,2-thiazine et de ses sels acceptables pour l'usage pharmaceutique, caractérisé en ce que l'on fait réagir un composé de formule générale II

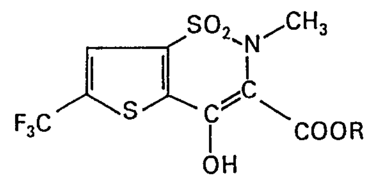

(II)

dans laquelle R représente un groupe alkyle en $C_1$–$C_7$, avec la 2-aminopyridine et, si on le désire, on convertit le composé ainsi obtenu en un sel acceptable pour l'usage pharmaceutique.

6. Médicament contenant du 1,1-dioxyde de la 4-hydroxy-2-méthyl-3-(2-pyridylcarbamoyl)-6-trifluorométhyl-2H-thiéno[2,3-e]-1,2-thiazine ou un sel acceptable pour l'usage pharmaceutique de ce composé.

7. Agent anti-inflammatoire, analgésique et anti-rhumatismal contenant du 1,1-dioxyde de la 4-hydroxy-2-méthyl-3-(2-pyridylcarbamoyl)-6-trifluorométhyl-2H-thiéno[2,3-e]-1,2-thiazine ou un sel acceptable pour l'usage pharmaceutique de ce composé.

**Revendication pour l'Etat contractant: AT**

Procédé de préparation du 1,1-dioxyde de la 4-hydroxy-2-méthyl-3-(2-pyridylcarbamoyl)-6-trifluorométhyl-2H-thiéno[2,3-e]-1,2-thiazine de formule I

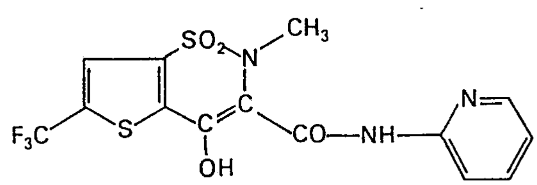

(I)

et de ses sels acceptables pour l'usage pharmaceutique, caractérisé en ce que l'on fait réagir un composé de formule générale II

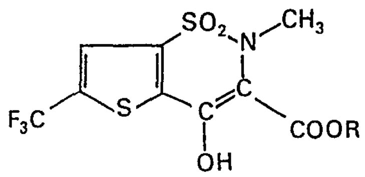

(II)

dans laquelle R représente un groupe alkyle en $C_1$–$C_7$, avec la 2-aminopyridine et, si on le désire, on convertit le composé ainsi obtenu en un sel acceptable pour l'usage pharmaceutique.